(19) Europäisches Patentamt
European Patent Office
Office européen des brevets

(11) EP 2 382 966 A1

(12) EUROPEAN PATENT APPLICATION

(43) Date of publication:
**02.11.2011 Bulletin 2011/44**

(51) Int Cl.:
***A61K 9/107*** *(2006.01)* ***A61K 9/51*** *(2006.01)*
***A61K 47/34*** *(2006.01)*

(21) Application number: **10156372.4**

(22) Date of filing: **12.03.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(71) Applicant: **DSM IP Assets B.V.**
**6411 TE Heerlen (NL)**

(72) Inventors:
- **Lebouille, Jérôme, George, Jozeph, Louis**
  **6100 AA Echt (NL)**
- **Kockelkoren, Tessa**
  **6100 AA Echt (NL)**
- **Vleugels, Leopold, Franciscus, Wijnandus**
  **6100 AA Echt (NL)**
- **Tuinier, Remco**
  **6100 AA Echt (NL)**

(74) Representative: **Vandevijver, Pascale**
**DSM Intellectual Property**
**P.O. Box 4**
**6100 AA Echt (NL)**

# (54) Micelle compositions and process for the preparation thereof

(57) The present invention relates to a micelle composition comprising a hydrophobic compound and an amphiphilic block copolymer, wherein the amphiphilic block copolymer consists of a hydrophobic block A and a hydrophilic block B, the hydrophobic block A comprises at least one hydrophobic polymeric unit X and the hydrophilic block B comprises at least one hydrophilic polymeric unit Y whereby the X and Y blocks alternate.

The present invention further relates to a process for the preparation of the micelle composition wherein the process comprises the steps of:
a) dissolving the hydrophobic compound and the amphiphilic block copolymer in an organic solvent to form a solution,
b) adding said organic solution into an aqueous medium,
c) optionally repeating aforementioned steps.

The micelle composition according to the present invention is useful in medical applications such as therapeutic cardiovascular applications, veterinary applications, food processing applications, flame retardant applications, coatings, adhesives and cosmetics, fabric/textiles, industrial and art applications.

EP 2 382 966 A1

## Description

The present invention relates to micelle compositions based on amphiphilic block copolymers. The present invention also relates to a process for the preparation of the micelle compositions suitable for medical and/or veterinary use. The invention also relates to articles or devices comprising the micelle composition.

The field of the present invention is the area of formulating hydrophobic compounds for use in aqueous systems, in particular, the formulation of relatively insoluble and/or toxic hydrophobic compounds such as cardiovascular drugs, anticancer agents, flavoring agents, vitamins, imaging agents, pigments, flame retardants, agricultural chemicals, fungicides, pesticides or insecticides.

Currently, potentially hydrophobic compounds have properties that can result in their classification as "challenging" (poorly-water-soluble) compounds. Such molecules have favorable in vitro capabilities, however due to characteristics such as poor aqueous solubility, toxicity, chemical instability, and limited cellular permeability, these compounds require formulation to be effective (Davis, S.S. et al. (1998) Int. J. Pharm. 179, 2).

Micelle systems based on amphiphilic block copolymers have been used to formulate such challenging compounds (Jones, M.C et al. (1999) Eur. J. Pharm. Biopharm. 48, 101). The amphiphilic block copolymers comprised of hydrophobic and hydrophilic blocks, can assemble into a microphase separated, core/shell architecture in a selective solvent. In an aqueous environment, the hydrophobic compound will be encapsulated into the hydrophobic core of the micelle while the aqueous solubility is provided by the shell of the micelle. Due to their nanoscopic dimensions and properties imparted by the shell, micelles may have long-term circulation capabilities.

WO-A-9710849 discloses biodegradable polymeric micelle-type drug compositions and method for the preparation of micelles comprising water insoluble drugs which micelles are composed of amphiphilic di- or tri-block copolymers containing poly(ethylene oxide) as hydrophilic block and poly(-ξ- caprolactone) as hydrophobic block. The molecular weight of the amphiphilic block copolymer used to form the micelles is in the range of about 1430 to 6000 Daltons. The resulting micelle-drug composition may be suitable for the sustained release of the water-insoluble drugs in vivo and this effect can be maximized by controlling the molecular weights and the relative ratio of the hydrophilic and hydrophobic blocks. WO-A-9710849 discloses different PLLA-PEO block copolymers and their water solubility's. The water solubility varies from 0.2 g/100 ml to over 20 g/100 ml. A disadvantage of these micelles, which are water soluble, is their tendency to aggregate so that the stability of the micelles on the longer term can not be assured.

WO-A-05118672 discloses micelles for the administration of hydrophobic drugs formed from self-assembly of poly (ethylene oxide)-b-poly (ξ-caprolactone) (PEO-b-PCL) block copolymers with a molecular weight above 6000 Dalton. It was found that the use of higher molecular weight block copolymers in the preparation of the micelles results in less aggregation of micelle particles and a modified biodistribution. This application is however silent about the stability and water solubility of the micelles.

There is a long felt need in the art for compositions for encapsulating poorly (water) soluble compounds for use in pharmaceutical, food, cosmetic and industrial formulations. Desirably the encapsulated materials are nanoscopic in size, thermodynamically and kinetically stable, protect the hydrophobic compounds from self-aggregation and provide advantageous release rates.

Therefore the object of the present invention is to provide a micelle composition comprising amphiphilic block copolymers which result in nanoscopic micelles which are thermodynamically and kinetically stable and which protect the hydrophobic compounds from self-aggregation and provide advantageous release properties.

The object of the present invention is achieved by providing a micelle composition comprising an amphiphilic block copolymer containing a hydrophobic block A and a hydrophilic block B, whereby the ratio R of the number average molecular weight ($M_n$) of block A ($M_n$ A) divided to the number average molecular weight of block B ($M_n$ B) is higher than 0.95 and whereby the amphiphilic block copolymer is characterised by a parameter $\alpha$ whereby

$$3 < \alpha < 5.5;$$

$$\alpha = M_A / (M_A+M_B) \times K_{o/w} \times \sqrt{}M_{tot}$$

in which

$M_A$ =molar mass of hydrophobic block(s) A
$M_B$ =molar mass of hydrophilic block(s) B
$K_{o/w}$ = Octanol/water partition coefficient

$$M_{tot} = M_A + M_B$$

Unexpectedly it has been found that micelles can be prepared with an optimum in the amount and the molecular weight of the hydrophilic/hydrophobic blocks A and B. It has surprisingly been found that stable micelles can be provided even on the longer term, whereby the tendency of the micelles to aggregate has been reduced markedly. Due to the stability of the micelle compositions, the micelles will exhibit enhanced properties on controlled release, shelf-life and exhibiting long circulation times in vivo. Moreover and at the same time the concentration of a drug in the micelle composition can be tailored to meet dosage needs. The micelle compositions of the present invention are capable of controlling the drug release. Such micelle compositions can offer several advantages over conventional dosage forms such as, decreased systemic side effects, and extended effective residence time of the drug, enhanced efficacy (targeted release) and patient's compliance, maintenance of therapeutic levels of the drug for longer time and with narrower fluctuations of drug's concentration in the plasma.

If $\alpha$ <3, the amphiphilic blockcopolymers become water soluble, which leads to aggregation and instable micelles. If $\alpha$ >5.5 the micelles become unstable.

The octanol/water partition coefficient (K) in parameter $\alpha$ is the ratio of the concentrations of a compound in the two phases of a mixture of two immiscible solvents at equilibrium. Hence these coefficients are a measure of differential solubility of the compound between these two solvents. Appropriate alternatives for the phrase "Partition Coefficient" are "partition constant", "partition ratio" or "distribution ratio". Normally one of the solvents chosen is water while the second solvent is hydrophobic for example octanol. Hence the partition coefficient is a measure of how "water loving" or "water fearing" a chemical substance is.

The amphiphilic blockcopolymer consists of a hydrophobic block A and a hydrophilic block B, the hydrophobic block A comprises at least one hydrophobic polymeric unit X and the hydrophilic block B comprises at least one hydrophilic polymeric unit Y whereby the X and Y units alternate. The hydrophobic polymeric units X and the hydrophilic polymeric units Y are preferably chosen such that the resulting amphiphilic block copolymer has a solubility in water $S_w$ of less than 0.1g/100 ml, more preferably less than 0.01 g/100ml, most preferably 0.001 g/100ml as determined by... The lower the solubility of the amphiphilic block copolymer in water, the more stable micelles can be prepared. The molecular weight of the amphiphilic block copolymer is chosen, at least in part, according to the size and flexibility of the hydrophobic compound.

The amphiphilic blockcopolymers are for example AB di-blocks, ABA-or BAB- tri-blockcopolymers but also multi-block copolymers having repeating BA or AB blocks to make A(BA)n or B(AB)n copolymers where n is an integer of from 2 to 5 are part of the present invention. Both ABA and BAB type triblock copolymers may be synthesized by ring opening polymerization, or condensation polymerization according to reaction schemes disclosed in US-A-5683723 and US-A-5702717, hereby fully incorporated by reference. For example they may be prepared via ring opening polymerization of one of the cyclic ester monomers, such as lactide, glycolide, or 1,4-dioxan-2-one with monomethoxy poly (ethylene glycol) (mPEG) or poly (ethylene glycol) (PEG) in the presence of stannous octoate as a catalyst at 80~130 Degrees C. The block copolymer product is dissolved in dichloromethane or acetone, precipitated in diethyl ether, hexane, pentane, or heptane, followed by drying.

The hydrophobic polymer units X may be chosen from the group consisting of polylactides, polycaprolactone, copolymers of lactide and glycolide, copolymers of lactide and caprolactone, copolymers of lactide and 1,4-dioxan-2-one, polyorthoesters, polyanhydrides, polyphosphazines, poly(hydroxybutyrate), poly(tetramethylene carbonate) or hydrophobic poly(ester amides), poly(amino acid)s or polycarbonates. Polymeric unit X is utilized because of its biodegradable, biocompatible, and solubilization properties. The *in vitro* and *in vivo* degradation of the hydrophobic, biodegradable polymer units X is well understood and the degradation products are naturally occurring compounds that are readily metabolized and/or eliminated by the patient's body. Preferably, hydrophobic polymer unit X is chosen from the group consisting of polylactide, polycaprolactone, a copolymer of lactide and glycolide, a copolymer of lactide and caprolactone, and a copolymer of lactide and 1,4-dioxan-2-one. The molecular weight of the hydrophobic polymer unit X is preferably within the range of 500-20,000 Daltons, and more preferably within the range of 1,000~10,000 Daltons.

The hydrophilic polymer units Y are for example chosen from hydrophilic polyesteramide, polyvinylalcohol or polyethylene glycol (PEG). PEG is preferably chosen as the hydrophilic, water-soluble block because of its unique biocompatibility, nontoxicity, hydrophilicity, solubilization properties. Also PEG copolymers based on the L-amino acids can be used. Examples include, without limitation, poly(ethyleneglycol)-b-poly(beta -benzyl-L-glutamate), poly(ethylene glycol)-b-poly(L-lysine acid), polyethylene glycol)-b-poly(aspartic acid, poly(ethylene glycol)- b-poly(beta -benzyl-L-aspartate), and acyl esters of the foregoing block copolymers. The molecular weight of the polyalkylene glycol or its derivatives is preferably within the range of 200-20,000 Daltons and more preferably within the range of 1,000~15,000 Daltons. The content of the hydrophilic component is within the range of 40-80 wt percent, preferably 40-70 wt percent, based on the total weight of the block copolymer.

Most preferably the amphiphilic block copolymer it is a triblock copolymer. The triblock copolymer preferably comprises polylactic acid, a hydrophobic polyesteramide or polycaprolactone as polymeric unit X and preferably polyethyleneglycol, polyvinylalcohol or a hydrophilic polyesteramide as polymeric unit Y. Specific examples include, but are not limited to PLGA-PEG-PLGA, PCL-PEG-PCL or poly (L-amino acid)-PEG-poly (L-amino acid) polymers.

It was moreover found that it is possible to produce monomodal micelles compositions. This is however dependent on the water solubility $S_w$ of the amphiphilic blockcopolymer. It has been found that monomodal micelle compositions can be prepared if the amphiphilic block copolymer has a $S_w$ of less than 0.1g/100 ml, more preferably less than 0.01 g/100ml, most preferably 0.001 g/100ml as determined by...

In the context of the present invention the term of "monomodal micelle composition" refers to an unfiltered micelle composition.

In a preferred embodiment, the invention relates to monomodal micelle compositions comprising a hydrophobic compound and an amphiphilic block copolymer, wherein the amphiphilic block copolymer consists of a hydrophobic blocks A and hydrophilic blocks B whereby the block A consists of one and the same hydrophobic unit X and the hydrophilic block B consists of one hydrophilic polymeric block Y, whereby the X and Y blocks alternate as X-Y-X. The ratio R of the number average molecular weight ($M_n$) of block A ($M_n$ A) divided to the number average molecular weight of block B ($M_n$ B), is higher than 0.95. Preferably the R is higher than 1.3 more preferably higher than 1.7, even more preferably higher than 2, most preferably higher than 3, for example higher than 3.5.

The hydrophobic compound as used herein is a compound which is not freely soluble in water and which is encapsulated within the amphiphilic block copolymer according to the present invention. Examples of the hydrophobic compounds include hydrophobic drugs such as anticancer agents, antiinflammatory agents, antifungal agents, antiemetics, antihypertensive agents, sex hormones, and steroids. Typical examples of the hydrophobic drugs are: anticancer agents such as paclitaxel, camptothecin, doxorubicin, daunomycin, cisplatin, 5-fluorouracil, mitomycin, methotrexate, and etoposide; antiinflammatory agents such as indomethacin, ibuprofen, ketoprofen, flubiprofen, diclofenac, piroxicam, tenoxicam, naproxen, aspirin, and acetaminophen; antifungal agents such as itraconazole, ketoconazole, and amphotericin; sex hormons such as testosterone, estrogen, progestone, and estradiol; steroids such as dexamethasone, prednisolone, and triamcinolone; antihypertensive agents such as captopril, ramipril, terazosin, minoxidil, and parazosin; antiemetics such as ondansetron and granisetron; antibiotics such as penicillin's for example B-lactams, chloramphenicol, metronidazole and fusidic acid; cyclosporine and biphenyl dimethyl dicarboxylic acid. Other examples of hydrophobic compounds are food ingredients, vitamins, pigments, dyes, insect repellents, UV light absorbing compounds, catalysts, photo-/UV-stabilizers, fungicides, insecticides or flame retardants. In particular, the hydrophobic compound may be selected from the group of nutrients, drugs, pharmaceuticals, proteins and peptides, vaccines, genetic materials, (such as polynucleotides, oligonucleotides, plasmids, DNA and RNA), diagnostic agents, and imaging agents.

The hydrophobic compound may be capable of stimulating or suppressing a biological response. The hydrophobic compound may for example be chosen from growth factors (VEGF, FGF, MCP-1, PIGF, anti-inflammatory compounds, antithrombogenic compounds, anti-claudication drugs, anti-arrhythmic drugs, anti-atherosclerotic drugs, antihistamines, cancer drugs, vascular drugs, ophthalmic drugs, amino acids, vitamins, hormones, neurotransmitters, neurohormones, enzymes, signalling molecules and psychoactive medicaments.

More examples of hydrophobic drugs are neurological drugs (amphetamine, methylphenidate), alpha1 adrenoceptor antagonist (prazosin, terazosin, doxazosin, ketenserin, urapidil), alpha2 blockers (arginine, nitroglycerin), hypotensive (clonidine, methyldopa, moxonidine, hydralazine minoxidil), bradykinin, angiotensin receptor blockers (benazepril, captopril, cilazepril, enalapril, fosinopril, lisinopril, perindopril, quinapril, ramipril, trandolapril, zofenopril), angiotensin-1 blockers (candesartan, eprosartan, irbesartan, losartan, telmisartan, valsartan), endopeptidase (omapatrilate), beta2 agonists (acebutolol, atenolol, bisoprolol, celiprolol, esmodol, metoprolol, nebivolol, betaxolol), beta2 blockers (carvedilol, labetalol, oxprenolol, pindolol, propanolol) diuretic actives (chlortalidon, chlorothiazide, epitizide, hydrochlorthiazide, indapamide, amiloride, triamterene), calcium channel blockers (amlodipin, barnidipin, diltiazem, felodipin, isradipin, lacidipin, lercanidipin, nicardipin, nifedipin, nimodipin, nitrendipin, verapamil), anti arthymic active (amiodarone, solatol, diclofenac, enalapril, flecainide) or ciprofloxacin, latanoprost, flucloxacillin, rapamycin and analogues and limus derivatives, paclitaxel, taxol, cyclosporine, heparin, corticosteroids (triamcinolone acetonide, dexamethasone, fluocinolone acetonide), anti-angiogenic (iRNA, VEGF antagonists: bevacizumab, ranibizumab, pegaptanib), growth factor, zinc finger transcription factor, triclosan, insulin, salbutamol, oestrogen, norcantharidin, microlidil analogues, prostaglandins, statins, chondroitinase, diketopiperazines, macrocycli compounds, neuregulins, osteopontin, alkaloids, immuno suppressants, antibodies, avidin, biotin, clonazepam.

The hydrophobic drugs can be delivered for local delivery or as pre or post surgical therapies for the management of pain, osteomyelitis, osteosarcoma, joint infection, macular degeneration, diabetic eye, diabetes mellitus, psoriasis, ulcers, atherosclerosis, claudication, thrombosis viral infection, cancer or in the treatment of hernia.

In the context of the present invention the term "micelle(s)" refers only to the amphiphilic block copolymers assembled into a microphase separated, core/shell architecture in a selective organic solvent. A micelle (plural micelles, micella, or micellae) is an aggregate of amphiphilic molecules dispersed in a liquid. A typical micelle in aqueous solution

forms an aggregate with the hydrophilic "head" regions in contact with surrounding solvent, sequestering the hydrophobic regions in the micelle centre. Micelles are approximately spherical in shape. Other phases, including shapes such as ellipsoids, cylinders, and rods are also possible. The shape and size of a micelle is a function of the molecular geometry of its molecules and solution conditions such as concentration, temperature, pH, and ionic strength.

The micelle composition according to the present invention may comprise a further hydrophobic core excipient such as a fatty acid, a vitamine or any hydrophobic polymer such as for example polycaprolactone. In this way the release properties can be further steered. Also the size of the micelles can be adjusted in this way.

The micelle composition of the present invention may optionally comprise a lyoprotectant. A lyoprotectant acts as a stabilizer for the loaded micelles during for example freeze drying. In this way the micelles do not coalesce so that the dried product does not readily disperse when an aqueous dispersant is added. The lyoprotectant can be a saccharide or polyol, for example, trehalose, sucrose or raffinose, or another hydrophilic polyol such as maltodextrin, fructose, glycerol, sorbitol, inositol and mannose. Lyoprotectants can also be materials other than sugars such as PEG.

Typically, the ratio of amphiphilic block copolymer to hydrophobic core excipient or lyoprotectant ranges from 1:1 w/w to about 1:50 w/w, preferably from 1:1 w/w to 1:10 w/w, advantageously to 1:5 w/w.

The lyoprotectant can be added to the solvent along with the hydrophobic compound and the amphiphilic block copolymer or it can be added to water upon bringing into water, the solution of the hydrophobic compound and the amphiphilic block copolymer formed in the organic solvent.

The micelles according to the present invention comprise an average particle size in the range of 10-800 nm, preferably 15-600 nm, more preferably 20-400 nm, most preferably in the range of 25-200 nm. The desired size is strongly dependent on the application and can be adjusted accordingly.

In general, micelles can be fabricated using a variety of techniques such as spray drying, freeze spray evaporation or emulsification (co-solvent evaporation). It is known to the person skilled in the art that the physical and chemical properties of micelles fabricated via emulsification, are greatly depended on the emulsification processing steps one applies for preparing the micelles. For example WO-A-03082303 discloses a process for the preparation of micelles which micelles comprise an amphiphilic block copolymers and a hydrophobic compound, and optionally a lyoprotectant or micelles' stabilizer. The process steps for producing the micelles include dissolving the hydrophobic compound and the amphiphilic block copolymer in a volatile organic solvent and then adding water to the miscible solution, with mixing, to promote the formation of micelles and the partitioning of the hydrophobic compound into the micelle cores. The water is added slowly to induce micellization through the critical water content of the amphiphilic block copolymers (level of water required for assembly of the amphiphilic block copolymers). The water content is greater than the critical weight concentration (CWC). Subsequently, the organic solvent is removed by evaporation under reduced pressure or elevated temperature. After loading, the micelles based on the amphiphilic block copolymers can be freeze dried for later reconstitution.

One of the main disadvantages is of this process is the sensivity to the amount and the addition rate of water up to the critical weight concentration (CWC), both being critical for the micelle formation and stability of the micelles but also for the end average particle size and particle size distribution.

Therefore it is a further object of the present invention to provide a process for the preparation of the micelles not having the above disadvantages.

The present invention further relates to a process for preparing the micelle composition wherein the process comprises the steps of:

a) dissolving the hydrophobic compound and the amphiphilic block copolymer in an organic solvent to form a solution,
b) adding said organic solution into an aqueous medium,
c) optionally repeating aforementioned steps.

In a preferred embodiment, the hydrophobic compound is a therapeutic agent.

The micelle composition may also comprise more than one hydrophobic compound.

The concentration of the amphiphilic block copolymer in the organic solvent depends on the organic solvent used. For example in case that acetone is used as a solvent the concentration of the amphiphilic block copolymer at most 130 mg/mL (milligram per litre), preferably is at most 100 mg/L, more preferably is at most 65 mg/L.

As used in the context of the present invention, an organic solvent is a water miscible liquid used to produce a solution with at least one amphiphilic block copolymer and at least one hydrophobic compound. For use in the present methods, the solvent is one which desirably has a boiling temperature lower than that of water (less than 100 degrees centigrade at 1 atm). Preferably, the organic solvent forms an azeotrope with water, advantageously a negative azeotrope. Where the solvent and water form an azeotrope, the azeoptropic mixture can be dried by removing the azeotrope under conditions of decreased pressure and/or elevated temperature. Examples include without limitation, acetone, methanol, ethanol, acetonitrile, tetrahydrofurane, propanol, isopropanol, ethyl acetate, etc.

In a preferred embodiment, the organic solvent is selected from the group consisting of acetone, tetrahydrofurane, methanol, ethanol, acetonitirile or mixtures thereof.

The aqueous medium is selected from the group consisting of water, saline solution or a buffer solution with a pH in the range of 1-14.

The process of the present invention offers enhanced control over the micelles' average particle size and distribution, the possibility to skip laborious and/or expensive process steps such as solvent evaporation, drying, sterilization, etc., Moreover the process is insensitive to the amount and/or the addition rate of water, it does not comprise a micelle's stabilizer such as a surfactant, it can be executed continuous on either small or large scale thus providing a robust, scalable and economically attractive method for the preparation of the micelle compositions.

The process of the present invention can also provide micelle compositions that can also exhibit one or more enhanced properties such as enhanced controlled release, enhanced self-life, being directly injectable and at the same time the concentration of the drug in the micelle composition can be tailored to meet dosage needs. Of course the process can be reversed to encapsulate hydrophilic compounds.

It is also possible to functionalize at least the surface of the micelles by providing at least the surface with a functional group, in particular with a signalling molecule, an enzyme or a receptor molecule, such as an antibody. The receptor molecule may for instance be a receptor molecule for a component of interest, which is to be purified or detected, e.g. as part of a diagnostic test, making use of the particles of the present invention. Suitable functionalisation methods may be based on a method known in the art.

In the context of the present invention the terms "method for the preparation" and "process" will be used interchangeably.

Unless the context clearly indicates otherwise, as used herein plural forms of the terms herein are to be construed as including the singular form and vice versa.

For all upper and lower boundaries of any parameters given herein, the boundary value is included in each range for each parameter. All combinations of minimum and maximum values of the parameters described herein may be used to define the parameter ranges for various embodiments and preferences of the invention.

It will be understood that the total sum of any quantities expressed herein as percentages cannot (allowing for rounding errors) exceed 100 %. For example the sum of all components of which the composition of the invention (or part(s) thereof) comprises may, when expressed as a weight (or other) percentage of the composition (or the same part (s) thereof), total 100 % allowing for rounding errors. However where a list of components is non exhaustive the sum of the percentage for each of such components may be less than 100 % to allow a certain percentage for additional amount (s) of any additional compound(s) that may not be explicitly described herein.

The micelle composition of the present invention can be administered, for example oral, parenteral, buccal, sublingual, nasal, rectal, patch, pump or transdermal administration and in pharmaceutical compositions formulated accordingly. Parenteral administration includes intravenous, infraperitoneal, subcutaneous, intramuscular, transepithelial, nasal, intrapulmonary, intrathecal, rectal and topical modes of administration. Parenteral administration may be by continuous infusion over a selected period of time. The micelles of the invention can be administered orally for example, with an inert diluent or with an assimilable edible carrier, it may be enclosed in hard or soft shell gelatin capsule, it may be compressed into tablets or it may be incorporated directly with the food of the diet. For oral therapeutic administration, the micelles of the present invention may be incorporated within an excipient and used in the form of ingestible tablets, buccal tablets, troches, capsules, elixirs, suspensions, syrups, wafers, and the like. The micelle composition of the invention may also be administered parenterally. Solutions of the micelle composition according to the present invention can be prepared in water. Under ordinary conditions of storage and use, these preparations contain a preservative to prevent the growth of microorganisms. A person skilled in the art would know how to prepare suitable formulations.

The fields wherein the micelles according to the present invention can be used include dermatology, vascular, orthopedics, ophthalmic, spinal, intestinal, pulmonary, nasal, or auricular. Besides in a pharmaceutical application, the micelles according to the present invention may inter alia be used in an agricultural or food application. In particular, such micelles may comprise food additives, pesticides, insecticides or plant-nutrients.

The present invention further relates to articles comprising the micelle composition of the present invention. In another aspect, the invention provides for a device comprising the micelle composition of the present invention. In the context of the present invention, an article is an individual object or item or element of a class designed to serve a purpose or perform a special function and can stand alone.

In yet another preferred embodiment, the invention provides for a device comprising the article of the present invention. A device is a piece of equipment or a mechanism designed to serve a special purpose or perform a special function and can consist of more than one article (multi-article assembly).

Examples of devices include, but are not limited to, drug eluting balloons, catheters, stents, rods, implants.

In another aspect the invention provides for the use of the micelle composition of the invention, the article of the invention, the device of the invention in medical applications such as therapeutic cardiovascular applications, veterinary applications, food processing applications, flame retardant applications, coatings, adhesives and cosmetics, fabric/textiles, industrial and art applications.

In another preferred embodiment, the invention provides for a micelle composition of the present invention for use as a medicament.

In yet another preferred embodiment, the invention provides for the use of a micelle composition of the present invention for the manufacture of a medicament for cardiovascular therapeutic applications.

In another preferred embodiment the invention provides for a method for manufacturing a medicament intended for cardiovascular therapeutic applications characterized in that the micelle composition of the present invention is used.

The present invention will now be described in detail with reference to the following non limiting examples which are by way of illustration only.

## EXAMPLES

### Materials and Methods.

PLGA 20kDa was purchased from Ingelheim Bhoeringer.
PCL 80kDa was purchased from Solvay
PEG (3.0kDa and 6.0kDa), dexamethasone and $Sn_2Oct$ were purchased from Sigma Aldrich.
Acetone was purchased from BASF.
Rapamycin was purchased from Oscar Tropitz.
Saline was purchased from BBraun.
Intensity-based Z-average as a particle size value measured by DLS
Polydispersity (PdI) is a measure of the width of the size distribution which is measured by the Malvern Zetasizer NanoZS

### Example 1: Preparation of PLGA-PEG-PLGA triblock copolymers via ring opening polymerization.

PEG was weighed into a two-necked round bottle flask after drying for 24 hours in a vacuum oven at 90°C and subsequently placed in an oil bath at 150°C. A vacuum was employed for at least 60 minutes before continuing synthesis. The addition of lactide and glycolide (molar ratio of lactyl:glycolyl= 50:50) was carried out by removing the vacuum and at the same time flushing with nitrogen gas. Under stirring a homogenous melt was obtained after which stannous octoate ($Sn_2Oct$), was added in the same way as the monomers. The reaction conditions were maintained for 20 hours where after the vacuum was replaced by nitrogen gas. The copolymers obtained in this way are listed below.

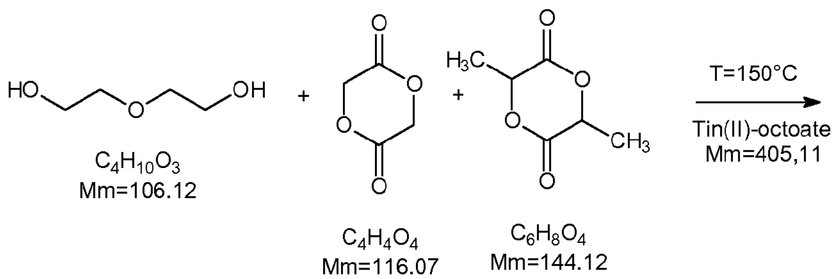


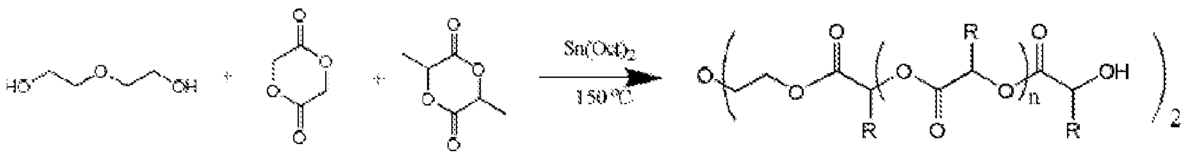


PEG-3000-diol
or
PEG-6000-diol

Batch 1: Synthesis of PLGA(50/50)-PEG-PLGA(50/50) 7.5k-6k-7.5k

| | Actual | Theory |
|---|---|---|
| - D,L-Lactide | 3.9131g | 3.96g |
| - Glycolide | 3.2471 g | 3.19g |
| - PEG-6000-diol | 2.8467g | 2.86g |
| - $Sn_2Oct$ | 2 drops | 4.4mg |

Batch 2: Synthesis of PLGA(50/50)-PEG-PLGA(50/50) 7.5k-6k-7.5k

| | Actual | Theory |
|---|---|---|
| - D,L-Lactide | 3.8461 g | 3.96g |
| - Glycolide | 3.1664g | 3.19g |
| - PEG-6000-diol | 2.8597g | 2.86g |
| - $Sn_2Oct$ | 2 drops | 4.4mg |

Batch 3: Synthesis of PLGA(50/50)-PEG-PLGA(50/50) 3.75k-3k-3.75k

| | Actual | Theory |
|---|---|---|
| - D,L-Lactide | 3.9098g | 3.96g |
| - Glycolide | 3.3233g | 3.19g |
| - PEG-3000-diol | 2.8573g | 2.86g |
| - $Sn_2Oct$ | 2 drops | 4.4mg |

Batch 4: Synthesis of PLGA(50/50)-PEG-PLGA(50/50) 3.75k-k-3.75k

| | Actual | Theory |
|---|---|---|
| - D,L-Lactide | 3.9790g | 3.96g |
| - Glycolide | 3.2620g | 3.19g |
| - PEG-3000-diol | 2.8548g | 2.86g |
| - $Sn_2Oct$ | 2 drops | 4.4mg |

<u>Example 2: Preparation of PCL-PEG-PCL triblock copolymers via ring opening polymerization.</u>

PEG was charged with ε-aprolactone in a 100ml round bottomed flask. The reaction mixture was heated to 100°C and stirred till a homogenous mixture was formed. A catalyst stock solution of tin(II)octoate (58.1 mg, 0.143 mmol) was prepared in hexane (5 mL). 1 mL of the catalyst stock solution was added to the reaction mixture at 100°C. The reaction mixture was further heated to 150°C for an additional 18 hours (overnight) to allow the reaction to proceed. The following morning the reaction mixture was cooled to room temperature, an off white waxy solid material was obtained.

Batch 1: Synthesis of PCL-PEG-PCL 1.5k-3.0k-1.5k

| | Actual | Theory |
|---|---|---|
| - ε-caprolactone | 14.9980 g | 0.131 mol |
| - PEG-3000-diol | 15.0004 g | 5.0 mmol |

(continued)

| | Actual | Theory |
|---|---|---|
| $Sn_2Oct$-hexane solution (1 mL used in synthesis) | | |
| - $Sn_2Oct$ | 58.1 mg | 0.143 mmol |
| - Hexane | | 5mL |

Batch 2: Synthesis of PCL-PEG-PCL 1.7k-3.0k-1.7k

| | Actual | Theory |
|---|---|---|
| - ε-caprolactone | 15.9433 g | 0.137 mol |
| - PEG-3000-diol | 14.0628 g | 4.7 mmol |
| $Sn_2Oct$-hexane solution (1 mL used in synthesis) | | |
| - $Sn_2Oct$ | 58.1 mg | 0.143 mmol |
| - Hexane | | 5mL |

Example 3: Purification of the synthesized triblock copolymers.

The triblock copolymers of examples 1 and 2 were dissolved in acetone at a weight percentage of 10-20% and filtered over an Acrodisc premium 25 mm Syringe filter, GxF/0.45μm PVDF membrane, to remove particulate impurities and dust particles, which can interfere with the nanoprecipitation process. Hereafter the filtered solution was collected into a beaker of 500 mL PTFE and evaporated to remove the solvent over night (10-12 hours) at maximum 40°C and minimum 300mmbar.

The blockcopolymers were characterized by $^1$H-NMR and GPC see Table 1.

Table 1: Tri-block copolymers composition

| PLGA-block | PEG-block | PLGA-block |
|---|---|---|
| 7.5kDa | 6kDa | 7.5kDa |
| 3.75kDa | 3kDa | 3.75kDa |
| 7.5kDa | 3kDa | 7.5kDa |
| **PCL-block** | **PEG-block** | **PCL-block** |
| 1.5kDa | 3kDa | 1.5kDa |
| 1.7kDa | 3kDa | 1.7kDa |
| 1.9kDa | 3kDa | 1.9kDa |
| 1.9kDa | 4kDa | 1.9kDa |
| 3.8kDa | 4kDa | 3.8kDa |
| 3.8kDa | 6kDa | 3.8kDa |

Example 4: Preparation of a micelle composition based on PLGA-PEG-PLGA triblockcopolymers

855 mg (PLGA 3.75k)$_2$-PEG 3k was dissolved in 14.25 ml Acetone selectipur. The solution was filtered over a 0.45 μm filter to remove dust particles.

183.85 mg Rapamycin and 183.85 mg PLGA-PTE 20k were dissolved in the (PLGA 3.75k)$_2$-PEG 3k / acetone solution. ([Rapa] = 12.9mg/ml).

The formulation was filtered over a 0.45 μm filter to remove dust particles.

1ml of the filtered formulation was pipetted into 25 ml of MilliQ water and measured by Dynamic light scattering (DLS).

Results and properties of the micelle composition are given in table 2.

Example 5: Preparation of a micelle composition based on PCL-PEG-PCL triblock copolymers

1439.14 mg (PCL2k)$_2$-PEG 3k was dissolved in Acetone selectipur (60 mg/ml). The polymer was filtered over

a 0.45 μm filter.

Rapamycin and PCL 80k was dissolved in the (PCL 2k)$_2$-PEG 3k /Acetone solution.The formulation was filtered over a 0.45 μm filter. 1 ml of the filtered formulation was pipetted into 25 ml of MilliQ water and measured by DLS.

Results and properties of the micelle composition are given in table 2.

Table 2

| Micelle composition | Shell material | Core material | | Z-average | PdI | Width | Distribution |
|---|---|---|---|---|---|---|---|
| Example 4 | (PLGA 3.75k)$_2$-PEG 3k | PLGA-PTE 20k 50% | Rapamicine 50% | 93.15 | 0.185 | 56.44 | Monomodal |
| Example 5 | (PCL 2k)$_2$-PEG 3k | PCL 80k 50% | Rapamicine 50% | 78.67 | 0.245 | 58.64 | Monomodal |

Example 6: Preparation of micelle compositions based on PLGA-PEG-PLGA

A. Micelles from different concentrations triblockcopolymer.

a. 32.1 mg PEG (6k) - (PLGA (7.5k))$_2$ was dissolved in 1ml acetone, 0.400ml of the solution was added to 10 ml Milli Q.
b. 64.5 mg PEG (6k) - (PLGA (7.5k))$_2$ was dissolved in 1ml acetone, 0.400ml of the solution was added to 10 ml Milli Q.

Results and properties of the micelle composition are given in table 3.

B. Micelles at different pH values

a. 64.5 mg PEG (6k) - (PLGA (7.5k))$_2$ was dissolved in 1ml acetone, 0.400ml of the solution was added to 10 ml pH buffer (CertiPUR buffer: citric acid / sodium hydroxide / hydrogen chloride), pH=4.
b. 64.5 mg PEG (6k) - (PLGA (7.5k))$_2$ was dissolved in 1ml acetone, 0.400ml of the solution was added to 10 ml pH buffer (CertiPUR buffer: boric acid/potassium chloride / sodium hydroxide), pH=9.

Results and properties of the micelle composition are given in table 3.

C. Micelles in different salt solution

a. 64.5 mg PEG (6k) - (PLGA (7.5k))$_2$ was dissolved in 1ml acetone, 0.400ml of the solution was added to 10 ml 0.9% NaCl.

Results and properties of the micelle composition are given in table 3.

D. Micelles in salt and pH controlled solution

a. 64.5 mg PEG (6k) - (PLGA (7.5k))$_2$ was dissolved in 1ml acetone, 0.400ml of the solution was added to 10 ml PBS, pH=7.4 (=sodium chloride / potassium chloride / sodium phosphate)

Results and properties of the micelle composition are given in table 3.

Table 3: Properties of the micelle compositions

| MIcelles | Aa | Ab | Ba | Bb | C | D |
|---|---|---|---|---|---|---|
| Z-average (nm) | 42.96 | 52.49 | 92.23 | 58.5 | 49.09 | 58.38 |
| PdI | 0.134 | 0.187 | 0.387 | 0.162 | 0.193 | 0.167 |
| Width (nm) | 19.7 | 32.02 | 110.1 | 24.83 | 11.71 | 29.09 |
| Distribution | Mono | Mono | Mono | Mono | Mono | Mono |
| Measuring temperature (C ) | 25.0 | 25.0 | 25.0 | 25.0 | 25.0 | 25.0 |

Example 7: Size stability of micelle compositions in time.

164.1 mg (PLGA 7.5k)$_2$-PEG 6k was dissolved in 2.400ml Acetone selectipur. The solution was filtered over a 0.45 μm filter to remove dust particles.
0.8 mg Rapamycin was dissolved in 0.800ml acetone solution.
The formulation was filtered over a 0.45 μm filter to remove dust particles.
0.3000 ml of the (PLGA 7.5k)$_2$-PEG 6k-acetone solution was mixed with 0.100ml of the rapamycin-acetone solution, resulting in 0.400 ml of (PLGA 7.5k)$_2$-PEG 6k/Rapamycin-acetone solution

The 0.400ml of the of (PLGA 7.5k)$_2$-PEG 6k/Rapamycin-acetone solution was pipetted into 10 ml of MilliQ water and measured by Dynamic light scattering (DLS).

| Time (days) | z-average (nm) | PdI |
|---|---|---|
| Day 1: | 45.28 | 0.202 |
| Day 2: | 43.81 | 0.197 |
| Day 15: | 45.27 | 0.113 |

Example 8: Size stability of micelle compositions in time.

164.1 mg (PLGA 7.5k)$_2$-PEG 6k was dissolved in 2.400ml Acetone selectipur. The solution was filtered over a 0.45 μm filter to remove dust particles.
0.3000 ml of the (PLGA 7.5k)$_2$-PEG 6k-acetone solution was mixed with 0.100ml of aacetone solution, resulting in 0.400 ml of (PLGA 7.5k)$_2$-PEG 6k-acetone solution

The 0.400ml of the of (PLGA 7.5k)$_2$-PEG 6k-acetone solution was pipetted into 10 ml of MilliQ water and measured by Dynamic light scattering (DLS).

| Time (days) | z-average (nm) | PdI |
|---|---|---|
| Day 1: | 48.69 | 0.188 |
| Day 15: | 49.08 | 0.100 |

**Claims**

**1.** A micelle composition comprising comprising an amphiphilic block copolymer containing a hydrophobic block A and a hydrophilic block B, whereby the ratio R of the number average molecular weight ($M_n$) of block A ($M_n$ A) divided to the number average molecular weight of block B ($M_n$ B) is higher than 0.95 and whereby the amphiphilic block copolymer is **characterised by** a parameter α whereby;

$$3 < \alpha < 5.5;$$

$$\alpha = M_A / (M_A+M_B) \times K_{o/w} \times \sqrt{M_{tot}}$$

in which

$M_A$ =molar mass of hydrophobic block(s) A
$M_B$ =molar mass of hydrophilic block(s) B
$K_{o/w}$= Octanol/water partition coefficient

$$M_{tot} = M_A + M_B$$

**2.** Micelle composition according to claim 1, wherein the amphiphilic block copolymer has solubility in water of less than 0.1 g/100 ml.

**3.** Micelle composition according to claim 1 or 2 wherein the hydrophobic block A comprises at least one hydrophobic polymeric unit X and the hydrophilic block B comprises at least one hydrophilic polymeric unit Y.

**4.** Micelle composition according to any one of the claims 1-3 further comprising a hydrophobic compound.

**5.** Micelle composition according to any one of the claims 1-4 wherein the average particle size of the micelles is in the range of 10-200 nm.

**6.** Micelle composition according to anyone of the claims 4-5 wherein the hydrophobic compound is selected from the group of therapeutic agents, cardiovascular drugs, vitamins, flavour agents, food ingredients, pigments, catalysts, photo-or UV-stabilizers, fungicides, insecticides, flame retardants or anticancer drugs.

**7.** Micelle composition according to claim 6 wherein the hydrophobic compound is a cardiovascular drug.

**8.** Micelle composition according to anyone of the claims 1-6 wherein the hydrophobic polymeric unit X are selected from the group consisting of poly(lactic acid), poly(D,L-lactide-co-glycolide), poly(∊-caprolactone), poly(hydroxybutyrate), poly(tetramethylene carbonate) or poly(ester amides).

**9.** Micelle composition according to anyone of the claims 1-7, wherein the hydrophilic polymeric unit Y are selected from the group consisting of poly(ethylene oxide), poly(ester amide), polyvinylpyrrolidone or polyvinylacetate.

**10.** Micelle composition according to anyone of the claims 1-9, wherein the amphiphilic block copolymer is a triblock copolymer comprising X-Y-X.

**11.** Micelle composition according to claim 10 wherein the triblock copolymer comprises polylactic acid, a hydrophobic polyesteramide or polycaprolactone as polymeric block X and polyethyleneglycol or a hydrophilic polyesteramide as polymeric block Y.

**12.** Micelle composition according to any one of the claims 1-11 wherein the micelle composition may further comprise a further hydrophobic core excipient.

**13.** A process for the preparation of the micelle composition according to any one of the claims 1-12 wherein the process comprises the steps of:

a. dissolving the hydrophobic compound and the amphiphilic block copolymer in an organic solvent to form a solution,
b. adding said organic solution into an aqueous medium,
c. optionally repeating aforementioned steps.

**14.** A process according to claim 13 wherein the process further comprises the following steps:

d) evaporating the organic solvent thus forming an aqueous solution,
e) optionally repeating aforementioned step with the steps described in claim 13.
f) filtering said aqueous solution to obtain the micelle composition,
g) optionally drying said micelles.

**15.** A process according to any one of claims 13-14 wherein the aqueous medium is selected from the group consisting

of water, saline solution or a buffer solution with a pH in the range of 1-14.

**16.** A process according to any one of claims 13-14 wherein the organic solvent is selected from the group consisting of acetone, tetrahydrofuran, methanol, ethanol, acetonitirile or mixtures thereof.

**17.** An article comprising the micelle composition according to any one of the claims 1-12.

**18.** A device comprising the micelle composition according to any one of the claims 1-12.

**19.** A device comprising the article of claim 17.

**20.** Use of the micelle composition according to any one of the claims 1-12, the article according to claim 17 or the device according to any one of the claims 18-19 in medical applications such as therapeutic cardiovascular applications, veterinary applications, food processing applications, flame retardancy applications, coatings, adhesives and cosmetics, fabric/textiles, industrial and art applications.

**21.** Use of the micelle composition according to claim 20 wherein the micelle composition is used in an amount that allows the micelle composition to exhibit its controlled release properties.

**22.** A micelle composition according to any one of the claims 1-12 for use as a medicament.

**23.** Use of a micelle composition as defined in any one of the claims 1-12 for the manufacture of a medicament for cardiovascular applications.

Europäisches Patentamt
European Patent Office
Office européen des brevets

# EUROPEAN SEARCH REPORT

Application Number
EP 10 15 6372

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/18142 A1 (MACROMED INC [US]) 15 April 1999 (1999-04-15) * claims 1-74; examples 1-10 * | 1-23 | INV. A61K9/107 A61K9/51 A61K47/34 |
| X | WO 02/076431 A1 (MACROMED INC [US]) 3 October 2002 (2002-10-03) * claims 1-13; examples 1-8 * | 1-23 | |
| X | WO 2005/058279 A1 (MACROMED INC [US]; SHIH CHUNG [US]; ZENTNER GAYLEN [US]; PIAO AL-ZHI [) 30 June 2005 (2005-06-30) * claims 1-12; examples 1-9 * | 1-23 | |
| X,D | WO 2005/118672 A1 (UNIV ALBERTA [CA]; LAVASANIFAR AFSANEH [CA]; KWON GLEN S [US]) 15 December 2005 (2005-12-15) | 1-9, 12-23 | |
| A | * page 20, line 16 - page 49, line 6; claims 1-48; tables 1,2 * | 10,11 | |
| X | GYUN SHIN I ET AL: "Methoxy poly(ethylene glycol)/epsilon-caprolactone amphiphilic block copolymeric micelle containing indomethacin. - I. Preparation and characterization" JOURNAL OF CONTROLLED RELEASE, ELSEVIER, AMSTERDAM, NL LNKD- DOI:10.1016/S0168-3659(97)00164-8, vol. 51, no. 1, 23 January 1998 (1998-01-23), pages 1-11, XP004108272 ISSN: 0168-3659 | 1-9, 12-23 | TECHNICAL FIELDS SEARCHED (IPC) A61K |
| A | * page 2, column 2 * * page 3, column 1 * * page 5, column 2 - page 7, column 2 * | 10,11 | |
| X | US 6 451 346 B1 (SHAH SUBODH [US] ET AL) 17 September 2002 (2002-09-17) * example 3 * | 1 | |
| | -/-- | | |

The present search report has been drawn up for all claims

1

| Place of search | Date of completion of the search | Examiner |
|---|---|---|
| The Hague | 23 July 2010 | Konter, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 15 6372

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHEN,W-Y ET AL: "Effect of Block Size and Sequence on the Micellization of ABC Triblock Methacrylic Polyampholytes" MACROMOLECULES, vol. 28, 1995, pages 8604-8611, XP002593144<br>* page 8605, column 1 - column 2 *<br>* page 8607, column 2 *<br>* page 8610, column 1 - column 2 *<br>----- | 1 | |
| | | | TECHNICAL FIELDS SEARCHED (IPC) |

The present search report has been drawn up for all claims

| Place of search | Date of completion of the search | Examiner |
|---|---|---|
| The Hague | 23 July 2010 | Konter, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........
& : member of the same patent family, corresponding document

1

EPO FORM 1503 03.82 (P04C01)

## ANNEX TO THE EUROPEAN SEARCH REPORT ON EUROPEAN PATENT APPLICATION NO. EP 10 15 6372

This annex lists the patent family members relating to the patent documents cited in the above-mentioned European search report.
The members are as contained in the European Patent Office EDP file on
The European Patent Office is in no way liable for these particulars which are merely given for the purpose of information.

23-07-2010

| Patent document cited in search report | Publication date | Patent family member(s) | Publication date |
|---|---|---|---|
| WO 9918142 A1 | 15-04-1999 | AT 290037 T | 15-03-2005 |
| | | AU 736812 B2 | 02-08-2001 |
| | | AU 9678098 A | 27-04-1999 |
| | | BR 9815239 A | 11-12-2001 |
| | | CA 2305621 A1 | 15-04-1999 |
| | | CN 1282345 A | 31-01-2001 |
| | | DE 69829204 D1 | 07-04-2005 |
| | | DE 69829204 T2 | 15-12-2005 |
| | | EP 1034207 A1 | 13-09-2000 |
| | | ES 2239408 T3 | 16-09-2005 |
| | | HK 1031736 A1 | 27-05-2005 |
| | | IL 135415 A | 13-04-2008 |
| | | JP 2002516910 T | 11-06-2002 |
| | | PT 1034207 E | 29-07-2005 |
| | | TR 200000900 T2 | 21-08-2000 |
| | | US 6201072 B1 | 13-03-2001 |
| WO 02076431 A1 | 03-10-2002 | US 2004185101 A1 | 23-09-2004 |
| | | US 2002192286 A1 | 19-12-2002 |
| WO 2005058279 A1 | 30-06-2005 | AU 2004299018 A1 | 30-06-2005 |
| | | CN 1889930 A | 03-01-2007 |
| | | EP 1691784 A1 | 23-08-2006 |
| | | JP 2007513970 T | 31-05-2007 |
| | | KR 20060120217 A | 24-11-2006 |
| WO 2005118672 A1 | 15-12-2005 | CA 2580305 A1 | 15-12-2005 |
| | | US 2008038353 A1 | 14-02-2008 |
| US 6451346 B1 | 17-09-2002 | AT 244556 T | 15-07-2003 |
| | | AU 769347 B2 | 22-01-2004 |
| | | CA 2355657 A1 | 06-07-2000 |
| | | DE 69909519 D1 | 14-08-2003 |
| | | DE 69909519 T2 | 24-12-2003 |
| | | ES 2197711 T3 | 01-01-2004 |
| | | JP 2002533377 T | 08-10-2002 |
| | | PT 1143929 E | 28-11-2003 |
| | | TW 250028 B | 01-03-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9710849 A
- WO 05118672 A
- US 5683723 A
- US 5702717 A
- WO 03082303 A

### Non-patent literature cited in the description


- **DAVIS, S.S. et al.** *Int. J. Pharm.,* 1998, vol. 179, 2
- **JONES, M.C et al.** *Eur. J. Pharm. Biopharm.,* 1999, vol. 48, 101